# EUROPÄISCHE PATENTSCHRIFT

(11) **EP 1 909 919 B1**
(45) Veröffentlichungstag und Bekanntmachung des Hinweises auf die Patenterteilung: **23.07.2014**
(21) Anmeldenummer: 06724781.7
(22) Anmeldetag: 10.05.2006
(51) Int. Cl.: A61Q 19/04, A61K 8/49, A61K 8/97, A61K 8/35, A61K 8/44, A61K 8/55

(54) **FLAVONOIDE ALS SYNERGISTEN ZUR VERSTÄRKUNG DER WIRKUNG VON SELBSTBRÄUNUNGSSUBSTANZEN**
FLAVONOID IN THE FORM OF A SYNERGIST FOR ENHANCING A SELF-BROWNING SUBSTANCE EFFECT
FLAVONOIDES UTILISES COMME SYNERGISTES POUR RENFORCER L'EFFET DE SUBSTANCES AUTOBRONZANTES

(30) Priorität: 27.07.2005 DE 102005035683; 28.07.2005 US 702983 P; 09.12.2005 US 748588 P
(43) Veröffentlichungstag der Anmeldung: 16.04.2008
(73) Patentinhaber: Merck Patent GmbH, 64293 Darmstadt (DE)
(72) Erfinder: RUDOLPH, Thomas, 64291 Darmstadt (DE); BUCHHOLZ, Herwig, 60599 Frankfurt (DE); MEDUSKI, Jerzy, F-78112 Fourqueux (FR)
(86) Internationale Anmeldenummer: PCT/EP2006/004384
(87) Internationale Veröffentlichungsnummer: WO 2007/012356

(56) Entgegenhaltungen:
- EP-A- 0 671 159
- EP-A- 1 092 415
- EP-A- 1 477 159
- WO-A-99/66897
- FR-A- 2 831 439
- GB-A- 2 333 772
- US-A- 3 639 444
- US-A1- 2001 046 479
- US-A1- 2003 138 387
- US-A1- 2004 126 342
- US-A1- 2004 191 208
- US-A1- 2004 228 810
- US-B1- 6 406 682
- PATENT ABSTRACTS OF JAPAN Bd. 018, Nr. 061 (C-1160), 2. Februar 1994 (1994-02-02) -& JP 05 279225 A (SUNTORY LTD), 26. Oktober 1993 (1993-10-26)

## Beschreibung

Die vorliegende Erfindung betrifft die Verwendung von Flavonoiden, ausgewählt aus Troxensin Rutinsulfat, Quercetin, isoquercetin und/oder Rutin, als Synergisten zur Verstärkung der Wirkung der Selbstbräunungssubstanzen DHA und/oder Erythrulose in kosmetischen und dermatologischen Formulierungen sowie entsprechende neue Zubereitungen und deren Herstellung.

Der Trend weg von der vornehmen Blässe hin zur "gesunden, sportlich braunen Haut" ist seit Jahren ungebrochen. Um diese zu erzielen, setzen die Menschen ihre Haut der Sonnenstrahlung aus, da diese eine Pigmentierung im Sinne einer Melaninbildung hervorruft. Die UV-Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.
Den natürlichen Schutz vor den negativen Folgen der Sonnenstrahlung bietet die Bräunung (Pigmentierung) der Haut. Die Epidermis enthält in ihrer untersten Schicht, der Basalschicht, neben den Basalzellen einzelne pigmentbildende Zellen, die Melanocyten. Durch UV-Licht wird in diesen Zellen die Produktion von Melanin angeregt, das in die Kerantinocyten (Hornzellen) transportiert und dort als braune Hautfarbe sichtbar wird.
Diese von der Aminosäure Tyrosin ausgehende Pigmentbildung wird überwiegend durch UVB-Strahlung initiiert und als "indirekte Pigmentierung" bezeichnet. Ihre Entwicklung läuft über mehrere Tage; die so erhaltene Sonnenbräune besteht über einige Wochen.
Bei der Direkt-Pigmentierung", die mit der Sonnenbestrahlung einsetzt, werden vorwiegend farblose Melanin-Vorstufen durch UVA-Strahlung zu dunkel gefärbten Melanin oxidiert. Da diese Oxidierung reversibel ist, führt sie zu einer nur kurz anhaltenden Hautbräunung.
Eine künstliche Bräunung der Haut lässt sich äußerlich mit Hilfe von Schminke und oral durch Einnahme von Carotinoiden erzeugen.
Weitaus beliebter jedoch ist die künstliche Bräunung der Haut, welche sich durch Auftragen von sogenannten Selbstbräunern erzielen lässt, wie sie Gegenstand der vorliegenden Erfindung sind. Diese Verbindungen weisen als chemisches Strukturmerkmal Keto- bzw. Aldehydgruppen in Nachbarschaft zu Alkoholfunktionen auf. Diese Ketole bzw. Aldole gehören überwiegend zur Substanzklasse der Zucker. Eine besonders häufig eingesetzte Selbstbräunungssubstanz ist 1,3-Dihydroxyaceton (DHA).
Die Verbindungen können mit den Proteinen und Aminosäuren der Hornschicht der Haut im Sinne einer Maillard-Reaktion umgesetzt werden, wobei über einen noch nicht vollständig aufgeklärten Reaktionsweg Polymerisate entstehen, die der Haut einen bräunlichen Farbton verleihen. Diese Reaktion ist nach etwa 4 bis 6 Stunden abgeschlossen. Die so erzielte Bräune ist nicht abwaschbar und wird erst mit der normalen Hautabschuppung entfernt.

Aufgabe der vorliegenden Erfindung war dementsprechend, Nachteile des Standes der Technik zu überwinden und Möglichkeiten zu finden, kosmetische und dermatologische Selbstbräunungsformulierungen bereitzustellen, die eine stärkere und länger andauernde Wirkung zeigen als die bekannter Bräunungsmittel.

Die Verwendung von Flavonoiden in der Kosmetik bzw. Dermatologie ist an sich bekannt. So beschreibt die DE 19739349 die Verwendung von Troxerutin als Antioxidans oder Radikalfänger in kosmetischen und dermatologischen Zubereitungen.
Es ist ebenfalls bekannt, dass Triosen und Tetrosen, insbesondere DHA und Erythrulose als Selbstbräunungssubstanzen in der Kosmetik verwendet werden.

So beschreibt die EP 1 172 090 Hautfärbemittel enthaltend DHA und ein Flavyliumsalz. Flavyliumsalze gehören zur Gruppe der Anthocyanidine, die nicht Gegenstand der erfindungsgemäß verwendeten Flavonoide sind.

Aus EP 1 092 415 sind kosmetische und dermatologische Lichtschutz-formulierungen bekannt, die DHA enthalten.

Aus EP 1 277 461 sind kosmetische Formulierungen bekannt, die DHA als Selbstbräunungssubstanz enthalten.

Aus EP 1 477 159 A1 sind wasserhaltige, kosmetische Zubereitungen bekannt, die ein oder mehrere selbstbräunende Substanzen (wie DHA) und mindestens ein Schichtsilikat sowie weitere Hilfs- und Zusatzstoffe enthalten.

Aus WO 2005/004826 ist die Verwendung einer kosmetischen Zubereitung zur Farbangleichung von unterschiedlich pigmentierten Hautstellen bekannt, die mindestens eine selbstbräunende Substanz (wie DHA) enthält.

Es konnte nun überraschend festgestellt werden dass mindestens ein Flavonoid ausgewählt aus Troxentin, Rutinsulfat, Quercetin, Isoquercetin und/oder Rutin, als Synergist zur Verstärkung der Wirkung von Selbstbräunungssubstanzen ausgewählt aus DHA und Erythrulose in kosmetischen und dermatologischen Formulierungen verwendet werden kann. Der Begriff "Flavonoid" im Sinne dieser Erfindung umfasst Substanzen deren Flavangrundkörper keine positive Ladung aufweist.

Selbstbräunungssubstanzen sind:

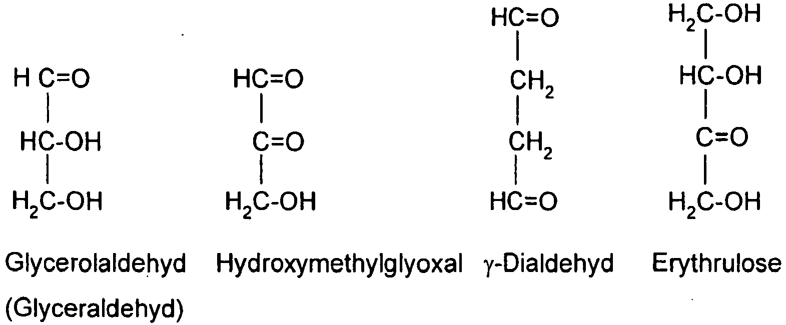

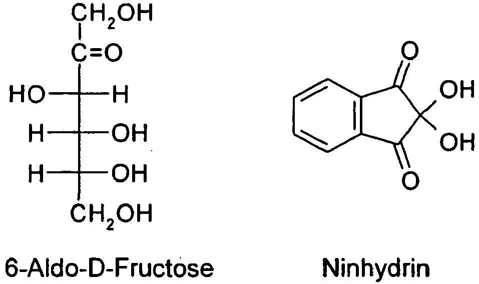

Ferner ist das 5-Hydroxy-1,4-naphtochinon (Juglon) zu nennen, das aus den Schalen frischer Walnüsse extrahiert wird, 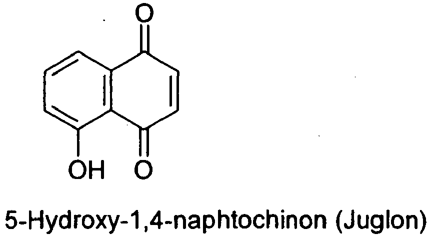 sowie das in den Henna-Blättern vorkommende 2-Hydroxy-1,4-naphtochinon (Lawson).

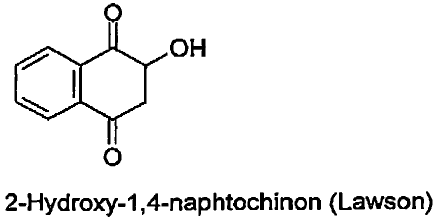

Weitere Selbstbräunungsaubtstanzen sind folgende Triosen und Tetrosen 1,3-Diyhydroxyaceton (DHA), Glyceraldehyd, Dihydroxyacetonphosphat, Glyceraldehydphosphat, Erythrose und 1,3,4-Trihydroxy-2-butanon (Erythrulose)..

Erfindungsgemäß eingesetzt wird Erythrulose und/oder 1,3-Dihydroxyaceton (DHA), ein im menschlichen Körper vorkommender dreiwertiger Ketozucker und dessen Derivate.

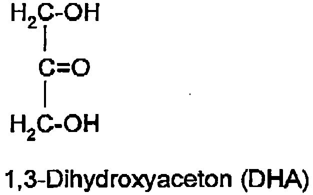

Alle Verbindungen oder Komponenten, die in den Zubereitungen verwendet werden können, sind entweder bekannt und käuflich erwerbbar oder können nach bekannten Verfahren synthetisiert werden.

Flavonoide lassen sich aufgrund ihrer Grundstruktur zu folgenden Gruppen zuordnen:
- Chalkone
- Aurone
- Flavanone
- Flavan-3-ole (Catechine)
- Flavone
- Isoflavone
- Flavan-3,4-diole (Leukoanthocyanidine)
- Flavonole (3-Hydroxy-flaven-4-on)
- Flavanonole

Der Name "Flavonoid" leitet sich vom lateinischen Wort flavus= gelb ab und berücksichtigt damit die Tatsache, dass die meisten dieser Substanzen in ihrer reinen Form eine gelbliche Farbe aufweisen.

Beispielhaft sollen folgende Flavonoide angeführt werden: 5-Hydroxy-7,4'-dimethoxyflavon-8-sulfat, 7,8-dihydroxyflavon, Luteolin (Flavone); Catechin, Epicatechin, EpiGalloCatechinGallat (EGCG, TEAVIGO^{®} DSM) (Flavan-3-ole bzw. Flavan-3-ol-derivate); Kämpferol (Flavonol); Taxifolin (Flavanonol).

Flavonoide kommen meist als lösliche Glykoside im Zellsaft der Pflanze vor. Die bevorzugten Flavonoide umfassen auch Aglyka (zuckerfreie Strukturen) und Aglyka-Konjugate. Mögliche Aglyka-Konjugate sind Hydroxyl-Derivate, wobei die Hydroxylgruppen ganz oder teilweise alkyliert, methyliert, glycyliert, sulfatiert oder verestert sind. Neben Hydroxylderivaten kommen auch C-Derivate als Aglyka-Konjugate in Frage.

Ein Mitglied der Gruppe der Flavonole ist das Agkykon Quercetin. Bei der Gruppe der Flavonol-o-glycoside sind die Flavonol-3-glycoside wie Rutin, α-Glucosylrutin, Tilirosid, Isoquercetin, Rutinsulfat, Trishydroxyethylrutin (Troxerutin) sowie deren Sulfaten und Phosphaten zu nennen. Bevorzugt sind Rutinsulfat und Troxerutin. Der Begriff "Rutinsulfat" umfasst Mono-, Di-, Tri-, Tetra- oder Polysulfate des Rutins bzw. Gemische dieser Rutinsulfate. Der Begriff "Troxerutin" umfasst Mono-, Di-, Tri- Tetra- oder Polyethoxylate des Rutins bzw. Gemische dieser Rutinethoxylate. Auch Flavonol-7- und -8-glycoside können verwendet werden.

Für die Gruppe der Flavonol- oder Flavonol-o-glycosid-enthaltenden Extrakte sind die Wirkstoffkombinationen Emblica, Licorice und/oder Rosskastanienextrakt zu nennen Emblica wird aus den Früchten des Laubbaumes Phyllanthus emblica (auch Emblica officinalis) z.B. in Indien, China, Pakistan oder Nepal gewonnen. Die Hauptinhaltsstoffe von Emblica sind die niedermolekularen Gerbsäuren Emblicanin A und B, die das in der Haut vorkommende Eisen in Form von Komplexen binden. Bevorzugte Emblica- Lösungen sind kommerziell z.B. als EMBLICA® (MERCK) oder CAPROS^{®} (siehe z.B. US-6,235,721 oder US-6,124,268) erhältlich. Prinzipiell kommen alle Emblica-Mischungen für eine Kombination mit den erfindungsgemäß einzusetzenden Selbstbräunungssubstanzen und Flavonoiden in Frage. Das Licorice-Extrakt enthält das Flavonoid Glabridin (ein Stearyl Glycyrhetinat). Das Rosskastanienextrakt enthält z.B. Esculin sowie weitere Flavonol- und/oder Flavonolglycosidbestandteile.

Solche pflanzlichen Substanzgemische können in dem Fachmann geläufiger Weise beispielsweise durch Auspressen oder Extrahieren der entsprechenden Pflanzen gewonnen werden.

Bevorzugt wird das Flavonoid in der kosmetischen oder dermatologischen Formulierung in einer Gesamtmenge von 0.01 bis 10 Gew.%, noch bevorzugter in einer Menge von 0.1 bis 5 Gew. % eingesetzt.

Bei der Bräunungs-Reaktion unterscheidet man, wie vorher schon erwährt, zwischen direkter und indirekter Reaktion. Die Bräunungs-Reaktion mittels Triosen und Tetrosen basiert auf einer direkten, nicht-enzymatischen Reaktion, welche analog zur bekannten Maillard-Reaktion abläuft. Sie wird durch UVA-Strahlung hervorgerufen und führt zu einer direkten Verdunklung der Hautpigmente. Dabei reagieren die genannten Substanzen als Ketosen mit Proteinen der Haut zu braungelben Farbprodukten, den sog. Melanoiden. Diese Farbprodukte besitzen allerdings selbst keine UV-absorbierenden Eigenschaften, sodass bei Sonnenexposition ein zusätzlicher Sonnenschutz (Bekleidung, Hut, UV-Filter) erforderlich wird.

Der indirekte Bräunungsprozess dagegen ist Enzym-gesteuert (durch das Enzym Tyrosinase) und wird durch UVB-Strahlung verursacht. Es ist weiterhin bekannt, dass einige chemische Wirkstoffe in der Lage sind, Tyrosinase zu aktivieren und somit indirekt die Melanin-Produktion erhöhen können. Kürzlich konnte dieser Effekt für Quercetin gezeigt werden (J. of Mol. Histology 35:157-165 (2004); Pigment Cell Res. 17: 66-73 (2004))

Durch Triose und/oder Tetrose direkt verursachte Bräunung geschieht schnell (innerhalb von Stunden), während indirekte Bräunung durch Quercetin einigeTage dauert.

Es war somit nicht vorhersehbar, dass Flavonoide (z.B. Quercetin) einen synergistischen Effekt bei der direkten Hautbräunung zeigen, wenn sie mit einer Triose (z.B. DHA) oder einer Tetrose (z.B. Erythrulose) kombiniert werden. Dieser vorteilhafte Effekt konnte durch folgenden Maillard-Test am Beispiel des DHA in Kombination mit Quercetin nachgewiesen werden:

Versuchslösungen von DHA mit Lysin mit oder ohne Quercetin wurden in einem Ethylenglycol-Wasser Gemisch zubereitet. Abhängig davon, ob Sauerstoff vorhanden war oder nicht, zeigte DHA unterschiedliches Reaktionsverhalten. In Abwesenheit von Sauerstoff reagierte DHA mit Lysin zu einer tiefbraunen Lösung. In Anwesenheit von Sauerstoff jedoch reagierte DHA mit gelber Farbe, es sei denn Quercetin war anwesend. Dann nämlich schlug die Farbe nach braun um. Es wird somit angekommen, dass Quercetin den schnellen Zerfall von DHA in Anwesenheit von Sauerstoff verhindern kann. Angewandte Maillard-Testsysteme sind in B. Nguyen et al. , J. Invest. Dermatol. 120 (2003), 655-661 beschrieben.

Die erfindungsgemäße Zubereitung, welche die Selbstbräunungssubstanz und das Flavonoid kombiniert, hat gegenüber einem Selbstbräunungsprodukt ohne Flavonoid-Zugabe folgende Vorteile:
- Stabilisierung der Triose bzw.Tetrose gegenüber Sauerstoff (auf der Haut und im Produkt)
- Beschleunigung der Bräunungs-Reaktion
- Verlängerung der Bräunungs-Reaktion aufgrund der indirekten Bräunungs-Reaktion (UV-freie Bräunungsverlängerung)
- Verstärkung der Bräunungs-Reaktion
- Erfindungsgemäße Zusammensetzung kommt der natürlichen Bräunung nahe

Alle erfindungsgemäß getesteten Flavonoide verfügen über einen nicht positiv geladenen Flavangrundkörper. Es ist zu vermuten, dass dies essentiell ist, um Metallionen wie z.B. Fe²⁺/Cu²⁺ auf der Haut ausreichend zu komplexieren. Dadurch bedingt wird die Selbstbräunungssubstanz vor Autooxidation geschützt. Demgegenüber ist ein positiv geladener Flavangrundkörper, wie ihn z.B. Anthocyanidine besitzen für diesen Zweck weniger geeignet.

Ein weiterer Gegenstand der vorliegenden Erfindung sind Zubereitungen, die mindestens ein Flavonoid ausgewählt aus Troxentin, Rutinsulfat, Quercetin und/oder Rutin und mindestens eine der Aktivsubstanzen Erythrulose und DHA enthalten, deren Wirkung durch die Anwesenheit des Flavonoids synergistisch verstärkt wird.

Möglich sind auch Zubereitungen, die Oxynex (wie z.B. Oxynex^{®} AP, Oxynex^{®} K LIQUID, Qxynex^{®} L LIQUID, Oxynex^{®} LM, Oxynex^{®} 2004, Oxynex ST (siehe WO 03/007908)) und eine Selbstbräunungssubstanz wie z.B. DHA enthalten.

Bei den Zubereitungen handelt es sich dabei üblicherweise um topisch anwendbare Zubereitungen, beispielsweise kosmetische oder dermatologische Formulierungen. Die Zubereitungen enthalten in diesem Fall einen kosmetisch oder dermatologisch geeigneten Träger und je nach gewünschtem Eigenschaftsprofil optional weitere geeignete Inhaltsstoffe.

Ein weiterer Gegenstand der Erfindung ist ein Verfahren zur Herstellung einer Zubereitung, wobei mindestens ein Flavonoid wie zuvor definiert sowie mindestens eine Aktivsubstanz wie zuvor definiert (z.B. Triose wie DHA) mit einem kosmetisch oder dermatologisch geeigneten Träger nacheinander auf der Haut vermischt bzw. appliziert werden. Vorzugsweise wird dieses Mischverfahren mit Hilfe eines Zweikammer-Flakon durchgeführt.

Bevorzugt ist ebenfalls ein Verfahren bei dem das Mischen bzw. die Applikation der erfindungsgemäßen Zubereitung auf der Haut dadurch geschieht, dass zunächst mit einer Flavonoid-Lösung geduscht wird und anschließend mit einer DHA-Lösung.

Darüber hinaus ist Gegenstand der vorliegenden Erfindung die Verwendung einer erfindungsgemäßen Zubereitung zur topischen Anwendung oder zur Anwendung auf einer Oberfläche.

Zu den einzusetzenden Substanzen zählen ferner z.B. UV-Filter, Flavon-Derivate, Chromon-Derivate, Aryloxime und Parabene.

Parabene sind 4-Hydroxybenzoesäureester, die in freier Form oder als NatriumSalze zur Konservierung von Zubereitungen im Bereich der Nahrungsmittel, Kosmetik und Arzneimittel verwendet werden. Die Wirkung der Ester ist direkt proportional zur Kettenlänge des Alkyl-Restes, umgekehrt nimmt jedoch die Löslichkeit mit steigender Kettenlänge ab. Als nicht dissoziierende Verbindungen sind die Ester weitgehend pH-Wert-unabhängig und wirken in einem pH-Bereich von 3,0-8,0. Der antimikrobielle Wirkmechanismus beruht auf einer Schädigung der Mikrobenmembranen durch die Oberflächenaktivität der PHB-Ester sowie auf der Eiweiß-Denaturierung. Daneben treten Interaktionen mit Coenzymen auf. Die Wirkung richtet sich gegen Pilze, Hefen und Bakterien. Die als Konservierungsmittel wichtigsten Parabene sind 4-Hydroxybenzoesäuremethylester, 4-Hydroxy-benzoesäureethylester, 4-Hydroxybenzoesäurepropylester, 4-Hydroxy-benzoesäurebutylester.

Unter den Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE 41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die neben den genannten Verbindung(en) zusätzlich ein Aryloxim, vorzugsweise 2-Hydroxy-5-methyllaurophenonoxim, enthalten, zeigen überraschende antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

Als Flavon-Derivate werden erfindungsgemäß Flavonoide und Coumaranone verstanden. Unter den Coumaranonen ist 4,6,3',4'-Tetrahydroxybenzyl-coumaranon-3 bevorzugt.

Unter Chromon-Derivaten werden vorzugsweise bestimmte Chromen-2-on-Derivate, die sich als Wirkstoffe zur vorbeugenden Behandlung von menschlicher Haut und menschlicher Haare gegen Alterungsprozesse und schädigende Umwelteinflüssen eignen, verstanden. Sie zeigen gleichzeitig ein niedriges Irritationspotential für die Haut, beeinflussen die Wasserbindung in der Haut positiv, erhalten oder erhöhen die Elastizität der Haut und fördern somit eine Glättung der Haut. Diese Verbindungen entsprechen vorzugsweise der folgenden Formel 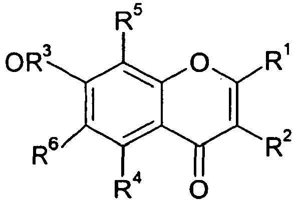 wobei
R¹ und R² gleich oder verschieden sein können und ausgewählt sind aus
- H, -C(=O)-R⁷, -C(=O)-OR⁷,
- geradkettigen oder verzweigten C₁- bis C₂₀-Alkylgruppen,
- geradkettigen oder verzweigten C₃- bis C₂₀-Alkenylgruppen, geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- C₃- bis C₁₀-Cycloalkylgruppen und/oder C₃- bis C₁₂-Cycloalkenylgruppen, wobei die Ringe jeweils auch durch -(CH₂)ₙ-Gruppen mit n = 1 bis 3 überbrückt sein können,
R³ steht für H oder geradkettige oder verzweigte C₁- bis C₂₀-Alkylgruppen,
R⁴ steht für H oder OR⁸,
R⁵ und R⁶ gleich oder verschieden sein können und ausgewählt sind aus
- -H, -OH,
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann und
R⁷ steht für H, geradkettige oder verzweigte C₁- bis C₂₀-Alkylgruppen, eine Polyhydroxy-Verbindung, wie vorzugsweise einen Ascorbinsäurerest oder glycosidische Reste und
R⁸ steht für H oder geradkettige oder verzweigte C₁- bis C₂₀-Alkylgruppen,
wobei mindestens 2 der Substituenten R¹, R², R⁴-R⁶ verschieden von H sind oder mindestens ein Substituent aus R¹ und R² für -C(=O)-R⁷ oder -C(=O)-OR⁷ steht.

Der Anteil an einer oder mehreren Verbindungen ausgewählt aus Flavonoiden, Chromon-Derivaten und Coumaranonen in der erfindungsgemäßen Zubereitung beträgt vorzugsweise von 0,001 bis 5 Gew.%, besonders bevorzugt von 0,01 bis 2 Gew.% bezogen auf die gesamte Zubereitung.

Die schützende Wirkung erfindungsgemäßer Zubereitungen gegen oxidativen Stress bzw. gegen die Einwirkung von Radikalen kann verbessert werden, wenn die Zubereitungen ein oder mehrere Antioxidantien enthalten, wobei es dem Fachmann keinerlei Schwierigkeiten bereitet geeignet schnell oder zeitverzögert wirkende Antioxidantien auszuwählen.

In einer bevorzugten Ausführungsform der vorliegenden Erfindung handelt es sich daher bei der erfindungsgemäßen Zubereitung um eine Zubereitung zum Schutz von Körperzellen gegen oxidativen Stress, insbesondere zur Verringerung der Hautalterung, dadurch gekennzeichnet, dass sie neben den Flavonoiden sowie den Selbstbräunungssubstanzen und gegebenenfalls anderen Inhaltsstoffen ein oder mehrere Antioxidantien enthält.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate; Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004). Derartige Antioxidantien werden mit den erfindungsgemäßen Verbindungen in solchen Zusammensetzungen üblicherweise in Verhältnissen im Bereich von 1000:1 bis 1:1000, bevorzugt in Mengen von 100:1 bis 1:100 eingesetzt.

Die erfindungsgemäß einzusetzenden Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Zubereitungen enthalten, insbesondere bevorzugt Vitamin-A-Palmitat, Vitamin C und dessen Derivate, DL-α-Tocopherol, Tocopherol-E-Acetat, Nicotinsäure, Pantothensäure und Biotin. Vitamine werden mit den erfindungsgemäßen Verbindungen in solchen Zusammensetzungen üblicherweise in Verhältnissen im Bereich von 1000:1 bis 1:1000, bevorzugt in Mengen von 100:1 bis 1:100 eingesetzt.

Unter den Phenolen mit antioxidativer Wirkung sind die teilweise als Naturstoffe vorkommenden Polyphenole für Anwendungen im pharmazeutischen, kosmetischen oder Ernährungsbereich besonders interessant. Beispielsweise weisen die hauptsächlich als Pflanzenfarbstoffe bekannten Flavonoide oder Bioflavonoide häufig ein antioxidantes Potential auf. Mit Effekten des Substitutionsmusters von Mono- und Dihydoxyflavonen beschäftigen sich K. Lemanska, H. Szymusiak, B. Tyrakowska, R. Zielinski, I.M.C.M. Rietjens; Current Topics in Biophysics 2000, 24(2), 101-108. Es wird dort beobachtet, dass Dihydroxyflavone mit einer OH-Gruppe benachbart zur Ketofunktion oder OH-Gruppen in 3'4'- oder 6,7- oder 7,8-Position antioxidative Eigenschaften aufweisen, während andere Mono- und Dihydroxyflavone teilweise keine antioxidativen Eigenschaften aufweisen.

Häufig wird Quercetin (Cyanidanol, Cyanidenolon 1522, Meletin, Sophoretin, Ericin, 3,3',4',5,7-Pentahydroxyflavon) als besonders wirksames Antioxidans genannt (z.B. C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers und I.M.C.M. Rietjens (Free Radical Biology&Medicine 2001, 31 (7), 869-881 untersuchen die pH-Abhängigkeit der antioxidanten Wirkung von Hydoxyflavonen. Über den gesamten pH-Bereich zeigt Quercetin die höchste Aktivität der untersuchten Strukturen.

Geeignete Antioxidantien sind ferner Verbindungen der Formel (III) 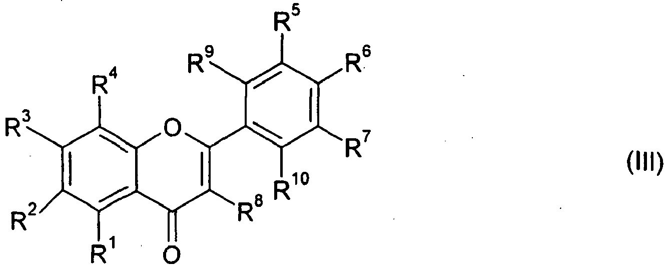 wobei R¹ bis R¹⁰ gleich oder verschieden sein können und ausgewählt sind aus
- H
- OR¹¹
- geradkettigen oder verzweigten C₁- bis C₂₀-Alkylgruppen,
- geradkettigen oder verzweigten C₃- bis C₂₀-Alkenylgruppen,
- geradkettigen oder verzweigten C₁- bis C₂₀-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- C₃- bis C₁₀-Cycloalkylgruppen und/oder C₃- bis C₁₂-Cycloalkenylgruppen, wobei die Ringe jeweils auch durch -(CH₂)ₙ-Gruppen mit n = 1 bis 3 überbrückt sein können,
- wobei alle OR¹¹ unabhängig voneinander stehen für
- OH
- geradkettige oder verzweigte C₁- bis C₂₀-Alkyloxygruppen,
- geradkettigen oder verzweigten C₃- bis C₂₀-Alkenyloxygruppen,
- geradkettigen oder verzweigten C₁- bis C₂₀-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder
- C₃- bis C₁₀-Cycloalkyloxygruppen und/oder C₃- bis C₁₂-Cycloalkenyloxygruppen, wobei die Ringe jeweils auch durch -(CH₂)ₙ-Gruppen mit n = 1 bis 3 überbrückt sein können und/oder,
- Mono- und/oder Oligoglycosylreste,
   mit der Maßgabe, dass mindestens 4 Reste aus R¹ bis R⁷ stehen für OH und dass im Molekül mindestens 2 Paare benachbarter Gruppen -OH vorliegen,
- oder R², R⁵ und R⁶ für OH und die Reste R¹, R³, R⁴ und R⁷⁻¹⁰ für H stehen, wie sie in der Deutschen Patentanmeldung DE-A-102 44 282 beschrieben sind.

Vorteile der erfindungsgemäßen Zusammensetzungen enthaltend mindestens ein Antioxidans sind dabei neben den oben genannten Vorteilen insbesondere die antioxidante Wirkung und die gute Hautverträglichkeit. Von Vorteil ist insbesondere das besondere Wirkprofil der Verbindungen nach Formel (III), welches sich im DPPH-Assay in einer hohen Kapazität Radikale zu fangen (EC₅₀), einer zeitverzögerten Wirkung (T_{EC50} > 120 min) und damit einer mittleren bis hohen antiradikalischen Effizienz (AE) äußert. Zudem vereinigen die Verbindungen nach Formel (III) im Molekül antioxidative Eigenschaften mit UV-Absorption im UV-A- und/oder -B-Bereich. Bevorzugt sind daher auch Zubereitungen enthaltend zumindest eine Verbindung der Formel (III), die dadurch gekennzeichnet ist, dass mindestens zwei benachbarte Reste der Reste R¹ bis R⁴ stehen für OH und mindestens zwei benachbarte Reste der Reste R⁵ bis R⁷ stehen für OH. Insbesondere bevorzugte Zubereitungen enthalten zumindest eine Verbindung der Formel (III), die dadurch gekennzeichnet ist, dass mindestens drei benachbarte Reste der Reste R¹ bis R⁴ für OH stehen, wobei vorzugsweise die Reste R¹ bis R³ für OH stehen.

Erfindungsgemäß insbesondere bevorzugte Zubereitungen können auch dem Sonnenschutz dienen und enthalten dann neben den Flavonoiden sowie den Selbstbräunungssubstanzen und gegebenenfalls anderen Inhaltsstoffen auch UV-Filter.

Prinzipiell kommen alle UV-Filter für eine Kombination mit den erfindungsgemäß einzusetzenden Selbstbräunungssubstanzen und Flavonoiden in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B. Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder (2-Oxoborn-3-yliden)toluol-4-sulfonsäure (z.B. Mexoryl® SL),
Benzoyl- oder Dibenzoylmethane wie 1-(4-tert-Butylphenyl)-3-(4-methoxyphenyl)-propan-1,3-dion (z.B. Eusolex® 9020) oder 4-Isopropyldibenzoylmethan (z.B. Eusolex® 8020),
Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),
Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),
Salicylatderivate wie 2-Ethylhexylsalicylat (z.B. Eusolex® OS), 4-Isopropylbenzyl-salicylat (z.B. Megasol®) oder 3,3,5-Trimethylcyclohexylsalicylat (z.B. Eusolex® HMS),
4-Aminobenzoesäure und Derivate wie 4-Aminobenzoesäure, 4-(Dimethylamino)-benzoesäure-2-ethylhexylester (z.B. Eusolex® 6007), ethoxylierter 4-Amino-benzoesäureethylester (z.B. Uvinul® P25),
Phenylbenzimidazolsulfonsäuren, wie 2-Phenylbenzimidazol-5-sulfonsäure sowie ihre Kalium-, Natrium- und Triethanolaminsalze (z.B. Eusolex® 232), 2,2-(1,4-Phenylen)-bisbenzimidazol-4,6-disulfonsäure bzw. deren Salze (z.B. Neoheliopan® AP) oder 2,2-(1,4-Phenylen)-bisbenzimidazol-6-sulfonsäure;
und weitere Substanzen wie
- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin (z.B. Uvinul® T 150)
- 2-(4-Diethylamino-2-hydroxy-benzoyl)-benzoesäure hexylester (z.B. Uvinul®UVA Plus, Fa. BASF).

Die in der Liste aufgeführten Verbindungen sind nur als Beispiele aufzufassen. Selbstverständlich können auch andere UV-Filter verwendet werden.

Weitere geeignete organische UV-Filter sind z.B.
- 2-(2H-Benzotriazol-2-yl)-4-methyl-6-(2-methyl-3-(1,3, 3,3-tetramethyl-1-(trimethylsilyloxy)disiloxanyl)propyl)phenol (z.B. Silatrizole^{®}),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),
- α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1)
- 2,2'-Methylen-bis-(6-(2H-benzotriazol-2-yl)-4-(1,1,3,3-tetramethylbutyl)phenol) (CAS-Nr. 103 597-45-1)
- 2,2'-(1,4-Phenylen)bis-(1H-benzimidazol-4,6-disulfonsäure, Mononatriumsalz) (CAS-Nr. 180 898-37-7) und
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),

Weitere geeignete UV-Filter sind auch Methoxyflavone ensprechend der älteren Deutschen Patentanmeldung DE-A-10232595.

Organische UV-Filter werden in der Regel in einer Menge von 0,5 bis 20 Gewichtsprozent, vorzugsweise 1 - 15 %, in kosmetische Formulierungen eingearbeitet.

Um einen optimierten UV-Schutz zu gewährleisten ist es weiter bevorzugt, wenn Zubereitungen mit Lichtschutzeigenschaften auch anorganische UV-Filter enthalten. Als anorganische UV-Filter sind solche aus der Gruppe der Titandioxide wie z.B. gecoatetes Titandioxid (z.B. Eusolex® T-2000, Eusolex^{®}T-AQUA, Eusolex® T-AVO), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Zubereitungen eingearbeitet.

Bevorzugte Verbindungen mit UV-filternden Eigenschaften sind 3-(4'-Methylbenzyliden)-dl-kampfer, 1-(4-tert-Butylphenyl)-3-(4-methoxy-phenyl)-pro-pan-1,3-dion, 4-Isopropyldibenzoylmethan, 2-Hydroxy-4-methoxy-benzophenon, Methoxyzimtsäureoctylester, 3,3,5-Trimethyl-cyclo-hexyl-sali-cylat, 4-(Dimethylamino)benzoesäure-2-ethyl-hexylester, 2-Cyano-3,3-di-phenyl-acrylsäure-2-ethylhexylester, 2-Phenyl-benzimidazol-5-sulfon-säure sowie ihre Kalium-, Natrium- und Triethanol-aminsalze.

Durch Kombination von einer oder mehrerer der genannten Verbindungen mit UV-Filterwirkung kann die Schutzwirkung gegen schädliche Einwirkungen der UV-Strahlung optimiert werden.

Optimierte Zusammensetzungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten. Mit dieser Kombination ergibt sich ein Breitbandschutz, der durch Zusatz von anorganischen UV-Filtern, wie Titandioxid-Mikropartikeln noch ergänzt werden kann.

Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:
- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.
- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.
- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.
- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher ist es erfindungsgemäß bevorzugt, wenn ein oder mehrere der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Silicium-oxid-hydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäß einzusetzenden Zubereitungen vorzugsweise in solchen Mengen enthalten, die gewährleisten, dass die verkapselten UV-Filter in den oben angegebenen Mengen in der Zubereitung vorliegen.

Die erfindungsgemäß einzusetzenden Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind beispielsweise auch sogenannte kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin- Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden: Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1- Diglycerin-Phosphat (DGP), β-Mannosylglycerat (Firoin), ß- Mannosylglyceramid (Firoin-A) oder/und Dimannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure) und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Ectoin und Ectoin-Derivate wie Hydroxyectoin können vorteilhaft in Arzneimitteln verwendet werden. Insbesondere kann Hydroxyectoin zur Herstellung eines Arzneimittels zur Behandlung von Hauterkrankungen eingesetzt werden. Andere Einsatzgebiete des Hydroxyectoins und anderer Ectoin-Derivate liegen typischerweise in Gebieten in denen z.B. Trehalose als Zusatzstoff verwendet wird. So können Ectoin-Derivate, wie Hydroxyectoin, als Schutzstoff in getrockneten Hefe- und Bakterienzellen Verwendung finden. Auch pharmazeutische Produkte wie nicht glykosylierte, pharmazeutische wirksame Peptide und Proteine z.B. t-PA können mit Ectoin oder seinen Derivaten geschützt werden.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-ups, Pflegecremes und Sonnenschutzpräparaten eingesetzt werden.

Dabei wird vorzugsweise eine Pyrimidincarbonsäure gemäß der folgenden Formel eingesetzt, 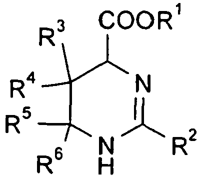 worin R¹ ein Rest H oder C1-8-Alkyl, R² ein Rest H oder C1-4-Alkyl und R³, R⁴, R⁵ sowie R⁶ jeweils unabhängig voneinander ein Rest aus der Gruppe H, OH, NH₂ und C1-4-Alkyl sind. Bevorzugt werden Pyrimidincarbonsäuren eingesetzt, bei denen R² eine Methyl- oder eine Ethylgruppe ist und R¹ bzw. R⁵ und R⁶ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäß einzusetzenden Zubereitungen derartige Pyrimidincarbonsäuren vorzugsweise in Mengen bis zu 15 Gew.-%.

Erfindungsgemäß insbesondere bevorzugt ist es dabei, wenn die kompatiblen Solute ausgewählt sind aus Di-myo-inositol-phosphat (DIP), cyclisches 2,3-diphosphoglycerat (cDPG), 1,1- Diglycerin-Phosphat (DGP), ß-Mannosylglycerat (Firoin), ß- Mannosylglyceramid (Firoin-A) oder/und Di-mannosyl-diinositolphosphat (DMIP), Ectoin, Hydroxyectoin oder Mischungen davon.

Unter den ebenfalls bevorzugt eingesetzten Aryloximen wird vorzugsweise 2-Hydroxy-5-methyllaurophenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die zusätzlich eine Aryloxim, vorzugsweise 2-Hydroxy-5-methyllaurophenonoxim enthalten, zeigen antiinflammatorische Eignung. Dabei enthalten die Zubereitungen vorzugsweise 0,01 bis 10 Gew.-% des Aryloxims, wobei es insbesondere bevorzugt ist, wenn die Zubereitung 0,05 bis 5 Gew-% Aryloxim enthält.

Die Flavonoide, die als Synergisten wirken, sowie die Selbstbräunungsmittel und gegebenenfalls weitere Wirkstoffe können in der üblichen Weise in kosmetische oder dermatologische Zubereitungen eingearbeitet werden. Geeignet sind die Zubereitungen bevorzugt für eine äußerliche Anwendung, beispielsweise als Creme, Lotion, Gel, oder als Lösung, die auf die Haut aufgesprüht werden kann. Für eine orale Anwendung wären Darreichungsformeln wie Kapseln, Dragees, Pulver, Tabletten-Lösungen oder Lösungen denkbar.

Als Anwendungsform der erfindungsgemäß einzusetzenden Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Bevorzugte Anwendungsformen sind Shampoos, Sonnenbäder und Duschbäder, die auch als sog. "Spray Tanning, Airbrush Tanning oder Sonnenduschen " aus kommerziellen Selbstbräunungs-Studios bekannt sind.

Spray Tanning, Airbrush Tanning oder Sonnendusche sind eigentlich gleich, d.h. man bringt z.B. eine DHA-Lösung (und weitere Aktivstoffe) auf den Körper auf. Der Unterschied liegt lediglich in der Ausführung.

Den Zubereitungen können beliebige übliche Trägerstoffe, Hilfsstoffe und gegebenenfalls weitere Wirkstoffe zugesetzt werden.

Ein weiterer Gegenstand der Erfindung ist ein Kit bestehend aus einem Zweikammer-System, vorzugsweise einem Zweikammer-Flakon, mit Pumpvorrichtung zur Aufbewahrung und Bereitstellung der erfindungsgemäßen Zusammensetzung nach Anspruch 1, wobei das Flavonoid und die Selbstbräunungssubstanz sowie weitere Aktiv- und Hilfsstoffe in zwei getrennten Kammern vorliegen und nacheinander auf die Haut appliziert werden. Da z.B. natürliche Vitamine in Cremegrundlagen oft instabil sind, könnten diese z.B. in einem Zweikammer-Flakon getrennt von der Cremegrundlage (z.B. eine DHA-Emulsion) aufbewahrt werden und erst auf der Haut direkt gemischt werden. Dadurch bleibt die Wirksamkeit empfindlicher Wirkstoffe (z.B. Vitamine) erhalten.

Bevorzugte Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.
Als weitere Anwendungsform ist das Zwei-Kammer-Flakon zu nennen, bei dem Bioflavonoid und Selbstbräunungssubstanz (z.B. DHA) in zwei getrennten Kammern aufbewahrt und durch mehrere Pumpstöße nacheinander auf die Haut gelangen und erst dort durch den Anwender gemischt werden. Dies hat den Vorteil, dass besonders empfindliche Wirkstoffe wie z.B. Vitamine, die in vielen Cremegrundlagen instabil sind, getrennt von einer Cremegrundlage aufbewahrt werden können und erst durch Betätigung des Spenders am Flakon der Cremegrundlage zugegeben werden. Dadurch bleibt die Wirksamkeit z.B. der Vitamine besser erhalten.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen leichtflüchtigen, verflüssigten Treibmittel, z.B. Chlorfluorkohlenwasserstoffe, Propan/Butan oder Dimethylether, enthalten. Auch Druckluft ist vorteilhaft zu verwenden.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestem, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenpflegestifte, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere auch Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:
- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Die wässrige Phase der erfindungsgemäß einzusetzenden Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Formulierung verwendet wird.

In einer bevorzugten Ausführungsform enthalten die erfindungsgemäß einzusetzenden Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren^{®} 1200 (Henkel KGaA), Oramix^{®} NS 10 (Seppic).

Die Acyllactylate werden ihrerseits vorteilhaft gewählt aus der Gruppe der Substanzen, welche sich durch die Strukturformel 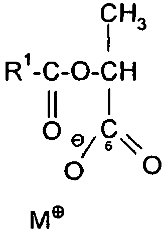 auszeichnen, wobei R¹ einen verzweigten oder unverzweigten Alkylrest mit 1 bis 30 Kohlenstoffatomen bedeutet und M⁺ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkaliions entspricht.

Vorteilhaft ist beispielsweise Natriumisostearyllactylat, beispielsweise das Produkt Pathionic^{®} ISL von der Gesellschaft American Ingredients Company.

Die Betaine werden vorteilhaft gewählt aus der Gruppe der Substanzen, welche sich durch die Strukturformel 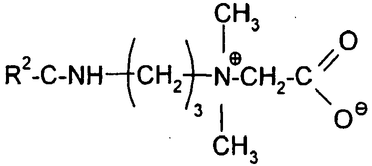 auszeichnen, wobei R² einen verzweigten oder unverzeigten Alkylrest mit 1 bis 30 Kohlenstoffatomen bedeutet.

Insbesondere vorteilhaft bedeutet R² einen verzweigten oder unverzweigten Alkylrest mit 6 bis 12 Kohlenstoffatomen.

Vorteilhaft ist beispielsweise Capramidopropylbetain, beispielsweise das Produkt Tego^{®} Betain 810 von der Gesellschaft Th. Goldschmidt AG.

Als erfindungsgemäß vorteilhaftes Cocoamphoacetat wird beispielsweise Natriumcocoamphoacetat gewählt, wie es unter der Bezeichnung Miranol^{®} Ultra C32 von der Gesellschaft Miranol Chemical Corp. erhältlich ist.

Die erfindungsgemäß einzusetzenden Zubereitungen sind vorteilhaft dadurch gekennzeichnet, dass das oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zusammensetzung, vorliegt oder vorliegen.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Wasser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-A-43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-Emulgatoren in den erfindungsgemäßen bevorzugten O/W-Emulsionen zu verwenden.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylen-glycol(17)stearylether (Steareth-17),Polyethylenglycol(18)stearylether (Steareth-18), Polyethylenglycol(19)stearylether (Steareth-19), Polyethylenglycol(20)-stearylether (Steareth-20), Polyethylenglycol(12)isostearylether (Isosteareth-12), Polyethylenglycol(13)isostearylether (Isosteareth-13), Polyethylenglycol(14)-isostearylether (Isosteareth-14), Polyethylenglycol(15)isostearylether (Isosteareth-15), Polyethylenglycol(16)isostearylether (Isosteareth-16), Polyethylenglycol(17)-isostearylether (Isosteareth-17), Polyethylenglycol(18)isostearylether (Isosteareth-18), Polyethylenglycol(19)isostearylether (Isosteareth-19), Polyethylenglycol(20)-isostearylether (Isosteareth-20), Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)-cetylether (Ceteth-17), Polyethylenglycol(18)cetylether (Ceteth-18), Polyethylen-glycol(19)cetylether (Ceteth-19), Polyethylen-glycol(20)cetylether (Ceteth-20), Polyethylenglycol(13)isocetylether (Isoceteth-13), Polyethylenglycol(14) - isocetylether (Isoceteth-14), Polyethylenglycol(15)isocetylether (Isoceteth-15), Polyethylenglycol(16)isocetylether (Isoceteth-16), Polyethylenglycol(17)-isocetylether (Isoceteth-17), Polyethylenglycol(18)isocetylether (Isoceteth-18), Polyethylenglycol(19)isocetylether (Isoceteth-19), Polyethylenglycol(20)-isocetylether (Isoceteth-20), Polyethylenglycol(12)oleylether (Oleth-12), Polyethylenglycol(13)oleylether (Oleth-13), Polyethylenglycol(14)oleylether (Oleth-. 14), Polyethylenglycol(15)oleylether (Oleth-15), Polyethylenglycol(12)laurylether (Laureth-12), Polyethylenglycol(12)isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)-cetylstearylether (Ceteareth-18), Polyethylenglycol(19)cetylstearylether (Ceteareth-19), Polyethylenglycol(20)cetylstearylether (Ceteareth-20).

Es ist ferner von Vorteil, die Fettsäureethoxylate aus folgender Gruppe zu wählen: Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat, Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat, Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat.

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Auch Polyethylenglycol-(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol(20)glyceryloleat, Polyethylenglycol(20)glycerylisostearat, Polyethylenglycol(18)glyceryloleat(cocoat) zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitan-monopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C--Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkohole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen liegen in verschiedenen, für diese Anwendung üblicherweise verwendeten Darreichungsformen vor. So können sie insbesondere als Lotion oder Emulsion, wie als Creme oder Milch (O/W, W/O, O/W/O, W/O/W), in Form ölig-alkoholischer, ölig-wässriger oder wässrigalkoholischer Gele bzw. Lösungen, als feste Stifte oder bevorzugt als Aerosol und Duschbad vorliegen.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -Milch vorliegt und beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfasst. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane , bevorzugt Alkane.

Weiterhin bevorzugt sind Zubereitungen, die lediglich aus einer Vormischung von Triose und/oder Tetrose sowie einem Flavonoid bestehen, wobei das Mischungsverhältnis zwischen 100 : 1 und 1 : 5 liegt. Besonders bevorzugt liegt das Mischungsverhältnis der Vormischung Triose zu Flavonoid zwischen 5 : 1 bis 1 : 2. Am bevorzugtesten ist eine Vormischung Triose (bevorzugt DHA) zu Flavonoid (bevorzugt Troxerutin) von 2 : 1.

Die erfindungsgemäß einzusetzenden Zubereitungen können dabei mit Hilfe von Techniken hergestellt werden, die dem Fachmann wohl bekannt sind.

Auch ohne weitere Ausführungen wird davon ausgegangen, dass ein Fachmann die obige Beschreibung in weitestem Umfang nutzen kann. Die bevorzugten Ausführungsformen sind deswegen lediglich als beschreibende, keinesfalls als in irgendeiner Weise limitierende Offenbarung aufzufassen.

Die im folgenden angeführten Beispiele für den erfindungsgemäßen Gegenstand dienen lediglich der Erläuterung und engen die vorliegende Erfindung keineswegs in irgendeiner Weise ein. Im übrigen ist die beschriebene Erfindung im gesamten beanspruchten Bereich ausführbar. Alle Verbindungen oder Komponenten, die in den Zubereitungen verwendet werden können, sind entweder bekannt und käuflich erhältlich oder können nach bekannten Methoden synthetisiert werden. Es werden die INCI-Namen der verwendeten Rohstoffe angegeben (die INCI-Namen werden definitionsgemäß in englischer Sprache angegeben).

### Beispiel 1: Bräunungsbeschleunigende in-vivo Wirkung von Troxerutin und Rutinsulfat bei DHA-Lösungen

| ITA° -Werte (Abnahme der ITA° -Werte im Vergleich zu t₀) | | | |
|---|---|---|---|
| | 2h | 4 h | 24 h |
| Formulierung A | -4.80 | -6.84 | -8.21 |
| Formulierung B | -4.98 | -6.99 | -8.35 |
| Formulierung C | -3.14 | -5.80 | -7.67 |

Die Formulierungen A, B, C enthalten jeweils 2% Dihydroxyaceton (DHA). Formulierung A enthält zusätzlich 1% Troxerutin, Formulierung B enthält zusätzlich 1% Rutinsulfat. Die Untersuchung wurde an 6 Probanden durchgeführt (Unterarm, Auftragsmenge 2mgcm⁻², Differenz zum unbehandelten Hautareal). Die Messungen der Hautfarbe wurden mit dem L*a*b*-System durchgefüht ("Commission Internationale de l'Eclairage" [CIE Publication, 1986]). Zur Auswertung der Chromametermessungen wurde insbesondere der ITA° -Wert herangezogen, der den visuellen Befund der Bräunungsverstärkung am besten widerspiegelte (ITA° -Wert = Individual Typologic Angle; ITA°= [ArcTan(L*-50)/b*)] x 180/π).

### Zubereitungen

Im folgenden werden beispielhaft Rezepturen für kosmetische Zubereitungen angegeben, die ein Flavonoid z.B. Rutin oder Troxerutin und eine Selbstbräunungssubstanz z.B. DHA enthalten. Im übrigen sind die INCI-Bezeichnungen der handelsüblichen Verbindungen angegeben. Die Herstellung der DHA-Duschlösungen erfolgt, indem man alle Rohstoffe einwiegt und homogen verrührt.

### Beispiel 2: DHA-Duschlösung

| **INCI** | **[%]** |
|---|---|
| Aqua (Water) | 75.05 |
| Dihydroxyacetone | 8.00 |
| Rutin | 0.75 |
| Ectoin | 0.30 |
| Propylene glycol | 4.50 |
| Glycerin | 2.00 |
| Ethoxydiglycol | 5.00 |
| Dimethyl isosorbide | 2.00 |
| Polysorbate 80 | 0.50 |
| Propylene glycol, walnut extract | 1.50 |
| Caramel | 0.10 |
| Parfum | 0.30 |

### Beispiel 3: DHA-Duschlösung

Zusammensetzung analog zu Beispiel 2 , nur anstelle von Rutin wird 0.75% Troxerutin eingesetzt.

### Beispiel 4: Selbstbräunungscreme mit Flavonoiden (O/W)

Die Herstellung der Selbstbräunungscreme erfolgt indem man Phase A (bestehend aus Glycerylstearat, Stearylalkohol, Ceterarylalkohol, Cetearylethylhexanoat, Capryltriglycerid, Stearoxydimethicon, Dimethicon, Tocopherylacetat, Propylparaben) und Phase B (bestehend aus Propylenglykol, Methylparaben und Wasser) erhitzt. Phase B wird langsam in Phase A eingerührt und homogenisiert. Unter Rühren wird abgekühlt. Das Rutin vor dem DHA im Wasser lösen. Bei 40 °C wird Phase C (bestehend aus DHA, Rutin und Wasser) zugegeben.

| **INCI** | **[%]** |
|---|---|
| Aqua (Water) | 69.00 |
| Dihydroxyacetone | 2.00 |
| Rutin | 1.00 |
| Methylparaben | 0.15 |
| Propylene glycol | 3.00 |
| Glyceryl stearate, Stearyl alcohol CETEH-20, STEARETH-25 | 8.00 |
| Cetearyl alcohol | 1.50 |
| Cetearyl ethylhexanoate | 6.50 |
| Caprylic/Capric triglyceride | 6.50 |
| Stearoxy dimethicone | 1.20 |
| Dimethicone | 0.50 |
| Tocopheryl acetate | 0.50 |
| Propylparaben | 0.05 |
| Parfum | 0.10 |

### Beispiel 5: Selbstbräunungscreme mit Flavonoiden (O/W)

Zusammensetzung analog zu Beispiel 4, nur anstelle von Rutin wird 1 % Troxerutin eingesetzt.

## Patentansprüche

1. Verwendung von Troxerutin, Rutinsulfat, Quercetin, Isoquercetin und/oder Rutin als Synergist zur Verstärkung der Wirkung von Dihydroxyaceton (DHA) und/oder 1,3,4-Trihydroxy-2-butanon (Erythrulose) in kosmetischen und dermatologischen Formulierungen, wobei das Verhältnis von DHA und/oder Erythrulose zu Troxerutin, Rutinsulfat, Quercetin, Isoquercetin und/oder Rutin zwischen 100:1 und 1:5 liegt.

2. Verwendung nach Anspruch 1, **dadurch gekennzeichnet, dass** Troxerutin und/oder Rutinsulfat als Synergist zur Verstärkung der Wirkung von DHA und/oder Erythrulose verwendet werden.

3. Verwendung nach Anspruch 1 und/oder 2, **dadurch gekennzeichnet, dass** das Verhältnis von DHA und/oder Erythrulose zu Troxerutin und/oder Rutinsulfat 2: 1 ist.

4. Verwendung nach einem oder mehreren der Ansprüche 1 bis 3, **dadurch gekennzeichnet, dass** die kosmetische oder dermatologische Formulierung mindestens ein Troxerutin und/oder Rutinsulfat, in einer Gesamtmenge von 0,1 bis 5 Gew.-%, enthält.

5. Verwendung nach mindestens einem oder mehreren der Ansprüche 1 bis 4, **dadurch gekennzeichnet, dass** die kosmetische oder dermatologische Formulierung mindestens eine Substanz enthält, die UV-Strahlung im UV-A- und/oder UV-B-Bereich absorbiert.

6. Verwendung nach mindestens einem oder mehreren der Ansprüche 1 bis 5, **dadurch gekennzeichnet, dass** die kosmetische oder dermatologische Formulierung anorganische Pigmente enthält, die als UV-Filtersubstanzen dienen.

7. Kosmetische und/oder dermatologische Zubereitung enthaltend Troxerutin, Rutinsulfat, Quercetin, Isoquercetin und/oder Rutin, und mindestens eine der Aktivsubstanzen Dihydroxyaceton und/oder Erythrulose, deren Wirkung durch die Anwesenheit von Troxerutin, Rutinsulfat, Quercetin, Isoquercetin und/oder Rutin synergistisch verstärkt wird, wobei das Verhältnis von DHA und/oder Erythrulose zu Troxerutin, Rutinsulfat, Quercetin, Isoquercetin und/oder Rutin zwischen 100:1 und 1:5 liegt.

8. Zubereitung nach Anspruch 7, **dadurch gekennzeichnet, dass** Troxerutin und/oder Rutinsulfat neben der mindestens einen Aktivsubstanz DHA und/oder Erythrulose eingesetzt werden.

9. Zubereitung nach Anspruch 7 und/oder 8, **dadurch gekennzeichnet, dass** das Verhältnis von DHA und/oder Erythrulose zu Troxerutin und/oder Rutinsulfat 2 : 1 ist.

10. Zubereitung nach einem oder mehreren der Ansprüche 7 bis 9, **dadurch gekennzeichnet, dass** die kosmetische oder dermatologische Formylierung Troxerutin und/oder Rutinsulfat, in einer Gesamtmenge von 0,1 bis 5 Gew.%, enthält.

11. Zubereitung nach einem oder mehreren der Ansprüche 7 bis 10, **dadurch gekennzeichnet, dass** sie weitere Aktivsubstanzen ausgewählt aus UV-Filtern, Chromon-Derivaten, Aryloxime und Parabenen enthält.

12. Zubereitung nach einem oder mehreren der Ansprüche 7 bis 11 zum Schutz von Körperzellen gegen oxidativen Stress, insbesondere zur Verringerung der Hautalterung, **dadurch gekennzeichnet, dass** sie ein oder mehrere weitere Antioxidantien und/oder Vitamine enthält.

13. Zubereitung nach einem oder mehreren der Ansprüche, 7 bis 12, wobei die Zubereitung neben Troxerutin, Rutinsulfat, Quercetin, Isoquercetin und/oder Rutin und der mindestens einen Aktivsubstanz Dihydroxyaceton und/oder Erythrulose, einen oder mehrere UV-Filter enthält, die ausgewählt sind aus 3-(4'-Methylbenzyliden)-dl-kampfer, 1-(4-tert-Butylphenyl)-3(4-methoxyphenyl)propan-1,3-dion, 2-Hydroxy-4-methoxybenzophenon, Methoxyzimtsäureethylhexylester, 3,3,5-Trimethyl-cyclohexylsalicylat, 4-(Dimethylamino)benzoesäure2-ethyl-hexylester, 2-Cyano-3,3-diphenylacrylsäure-2-ethylhexylester, 2-Phenylbenzimidazol-5-sulfonsäure und Kalium-, Natrium- und Triethanolaminsatze.

14. Zubereitung bestehend aus einer Vormischung aus Dihydroxyaceton und/oder Erythrulose sowie Troxerutin, Rutinsulfat, Quercetin, Isoquercetin und/oder Rutin im Verhältnis 100:1 bis 1:5.

15. Zubereitung nach Anspruch 14, **dadurch gekennzeichnet, dass** die Vormischung aus Dihydroxyaceton sowie Troxerutin und/oder Rutinsulfat im Verhältnis 100:1 bis 1:5 besteht.

16. Zubereitung nach Anspruch 14 und/oder 15, **dadurch gekennzeichnet, dass** das Verhältnis der Vormischung aus Dihydroxyaceton sowie Troxerutin und/oder Rutinsulfat 2 : 1 ist.

17. Verfahren zur Herstellung einer Zubereitung nach einem oder mehreren der Ansprüche 7 bis 13, **dadurch gekennzeichnet, dass** Troxerutin, Rutinsulfat, Quercetin, Isoquercetin und/oder Rutin sowie mindestens eine der Aktivsubstanzen Dihydroxyaceton und/oder Erythrulose mit einem kosmetisch oder dermatologisch geeigneten Träger nacheinander vermischt werden.

18. Nicht-therapeutische Verwendung einer Zubereitung nach einem oder mehreren der Ansprüche 7 bis 13 zur topischen Anwendung oder zur Anwendung auf einer Oberfläche

## Claims

1. Use of troxerutin, rutin sulfate, quercetin, isoquercetin and/or rutin as synergist for enhancing the action of dihydroxyacetone (DHA) and/or 1,3,4-tri-hydroxy-2-butanone (erythrulose) in cosmetic and dermatological formulations, where the ratio of DHA and/or erythrulose to troxerutin, rutin sulfate, quercetin, isoquercetin and/or rutin is between 100 : 1 and 1 : 5.

2. Use according to Claim 1, **characterised in that** troxerutin and/or rutin sulfate are used as synergist for enhancing the action of DHA and/or erythrulose.

3. Use according to Claim 1 and/or 2, **characterised in that** the ratio of DHA and/or erythrulose to troxerutin and/or rutin sulfate is 2 : 1.

4. Use according to one or more of Claims 1 to 3, **characterised in that** the cosmetic or dermatological formulation comprises at least one troxerutin and/or rutin sulfate, in a total amount of 0.1 to 5% by weight.

5. Use according to at least one or more of Claims 1 to 4, **characterised in that** the cosmetic or dermatological formulation comprises at least one substance which absorbs UV radiation in the UV-A and/or UV-B region.

6. Use according to at least one or more of Claims 1 to 5, **characterised in that** the cosmetic or dermatological formulation comprises inorganic pigments which serve as UV-filter substances.

7. Cosmetic and/or dermatological preparation comprising troxerutin, rutin sulfate, quercetin, isoquercetin and/or rutin, and at least one of the active substances dihydroxyacetone and/or erythrulose, whose action is synergistically enhanced by the presence of troxerutin, rutin sulfate, quercetin, isoquercetin and/or rutin, where the ratio of DHA and/or erythrulose to troxerutin, rutin sulfate, quercetin, isoquercetin and/or rutin is between 100 : 1 and 1 : 5.

8. Preparation according to Claim 7, **characterised in that** troxerutin and/or rutin sulfate are employed besides the at least one active substance DHA and/or erythrulose.

9. Preparation according to Claim 7 and/or 8, **characterised in that** the ratio of DHA and/or erythrulose to troxerutin and/or rutin sulfate is 2 : 1.

10. Preparation according to one or more of Claims 7 to 9, **characterised in that** the cosmetic or dermatological formulation comprises troxerutin and/or rutin sulfate, in a total amount of 0.1 to 5% by weight.

11. Preparation according to one or more of Claims 7 to 10, **characterised in that** it comprises further active substances selected from UV filters, chromone derivatives, aryl oximes and parabens.

12. Preparation according to one or more of Claims 7 to 11 for the protection of body cells against oxidative stress, in particular for reducing skin ageing, **characterised in that** it comprises one or more further antioxidants and/or vitamins.

13. Preparation according to one or more of Claims 7 to 12, where the preparation, besides troxerutin, rutin sulfate, quercetin, isoquercetin and/or rutin and the at least one active substance dihydroxyacetone and/or erythrulose, comprises one or more UV filters which are selected from 3-(4'-methyl-benzylidene)-dl-camphor, 1-(4-tert-butylphenyl)-3-(4-methoxyphenyl)propane-1,3-dione, 2-hydroxy-4-methoxybenzophenone, ethylhexyl methoxycinnamate, 3,3,5-trimethylcyclohexyl salicylate, 2-ethylhexyl 4-(dimethyl-amino)benzoate, 2-ethylhexyl 2-cyano-3,3-diphenylacrylate, 2-phenyl-benzimidazole-5-sulfonic acid and potassium, sodium and triethanolamine salts.

14. Preparation consisting of a pre-mix of dihydroxyacetone and/or erythrulose as well as troxerutin, rutin sulfate, quercetin, isoquercetin and/or rutin in the ratio 100 : 1 to 1 : 5.

15. Preparation according to Claim 14, **characterised in that** the pre-mix consists of dihydroxyacetone as well as troxerutin and/or rutin sulfate in the ratio 100 : 1 to 1 : 5.

16. Preparation according to Claim 14 and/or 15, **characterised in that** the ratio of the pre-mix of dihydroxyacetone as well as troxerutin and/or rutin sulfate is 2 : 1.

17. Process for the preparation of a preparation according to one or more of Claims 7 to 13, **characterised in that** troxerutin, rutin sulfate, quercetin, isoquercetin and/or rutin as well as at least one of the active substances dihydroxyacetone and/or erythrulose are mixed successively with a cosmetically or dermatologically suitable vehicle.

18. Non-therapeutic use of a preparation according to one or more of Claims 7 to 13 for topical application or for application to a surface.

## Revendications

1. Utilisation de troxérutine, de sulfate de rutine, de quercétine, d'isoquercétine et/ou de rutine comme agent synergique pour améliorer l'action de la dihydroxyacétone (DHA) et/ou de la 1,3,4-trihydroxy-2-butanone (érythrulose) dans des formulations cosmétiques et dermatologiques, **caractérisée en ce que** le rapport de la DHA et/ou de l'érythrulose à la troxérutine, au sulfate de rutine, à la quercétine, à l'isoquercétine et/ou à la rutine est compris entre 100:1 et 1:5.

2. Utilisation selon la revendication 1, **caractérisée en ce que** la troxérutine et/ou le sulfate de rutine sont utilisés comme agent synergique pour améliorer l'action de la DHA et/ou de l'érythrulose.

3. Utilisation selon la revendication 1 et/ou 2, **caractérisée en ce que** le rapport de la DHA et/ou de l'érythrulose à la troxérutine et/ou au sulfate de rutine est de 2:1.

4. Utilisation selon l'une ou plusieurs parmi les revendications 1 à 3, **caractérisée en ce que** la formulation cosmétique ou dermatologique comprend au moins un parmi la troxérutine et/ou le sulfate de rutine, selon une quantité totale allant de 0,1 à 5% en poids.

5. Utilisation selon au moins l'une ou plusieurs parmi les revendications 1 à 4, **caractérisée en ce que** la formulation cosmétique ou dermatologique comprend au moins une substance qui absorbe le rayonnement UV dans la région des UV-A et/ou des UV-B.

6. Utilisation selon au moins l'une ou plusieurs parmi les revendications 1 à 5, **caractérisée en ce que** la formulation cosmétique ou dermatologique comprend des pigments inorganiques qui servent de substances anti-UV.

7. Préparation cosmétique et/ou dermatologique comprenant de la troxérutine, du sulfate de rutine, de la quercétine, de l'isoquercétine et/ou de la rutine, et au moins une parmi les substances actives de dihydroxyacétone et/ou d'érythrulose, dont l'action est améliorée de manière synergique par la présence de troxérutine, de sulfate de rutine, de quercétine, d'isoquercétine et/ou de rutine, **caractérisée en ce que** le rapport de la DHA et/ou de l'érythrulose à la troxérutine, au sulfate de rutine, à la quercétine, à l'isoquercétine et/ou à la rutine est compris entre 100:1 et 1:5.

8. Préparation selon la revendication 7, **caractérisée en ce que** la troxérutine et/ou le sulfate de rutine sont employés outre la au moins une substance active de DHA et/ou d'érythrulose.

9. Préparation selon la revendication 7 et/ou 8, **caractérisée en ce que** le rapport de la DHA et/ou de l'érythrulose à la troxérutine et/ou au sulfate de rutine est de 2:1.

10. Préparation selon l'une ou plusieurs parmi les revendications 7 à 9, **caractérisée en ce que** la formulation cosmétique ou dermatologique comprend de la troxérutine et/ou du sulfate de rutine, selon une quantité totale allant de 0,1 à 5% en poids.

11. Préparation selon l'une ou plusieurs parmi les revendications 7 à 10, **caractérisée en ce qu'**elle comprend d'autres substances actives choisies parmi les filtres anti-UV, les dérivés de chromone, les aryl oximes et les parabènes.

12. Préparation selon l'une ou plusieurs parmi les revendications 7 à 11, pour la protection de cellules corporelles contre un stress oxydatif, en particulier pour réduire le vieillissement de la peau, **caractérisée en ce qu'**elle comprend un ou plusieurs autres parmi des antioxydants et/ou des vitamines.

13. Préparation selon l'une ou plusieurs parmi les revendications 7 à 12, **caractérisée en ce que** la préparation, outre la troxérutine, le sulfate de rutine, la quercétine, l'isoquercétine et/ou la rutine et la au moins une substance active de dihydroxyacétone et/ou d'érythrulose, comprend un ou plusieurs filtres anti-UV qui sont choisis parmi le 3-(4'-méthylbenzylidène)-dl-camphre, la 1-(4-tertio-butylphényl)-3-(4-méthoxyphényl)propane-1,3-dione, la 2-hydroxy-4-méthoxybenzophénone, le méthoxycinnamate d'éthylhexyle, le salicylate de 3,3,5-triméthylcyclohexyle, le 4-(diméthylamino)benzoate de 2-éthylhexyle, le 2-cyano-3,3-diphénylacrylate de 2-éthylhexyle, l'acide 2-phénylbenzimidazole-5-sulfonique et les sels de potassium, sodium et triéthanolamine.

14. Préparation constituée d'un prémélange de dihydroxyacétone et/ou d'érythrulose ainsi que de troxérutine, de sulfate de rutine, de quercétine, d'isoquercétine et/ou de rutine selon un rapport allant de 100:1 à 1:5.

15. Préparation selon la revendication 14, **caractérisée en ce que** le prémélange est constitué de dihydroxyacétone ainsi que de troxérutine et/ou de sulfate de rutine selon un rapport allant de 100:1 à 1:5.

16. Préparation selon la revendication 14 et/ou 15, **caractérisée en ce que** le rapport du prémélange de dihydroxyacétone ainsi que de troxérutine et/ou de sulfate de rutine est de 2:1.

17. Procédé de préparation d'une préparation selon l'une ou plusieurs parmi les revendications 7 à 13, **caractérisé en ce que** la troxérutine, le sulfate de rutine, la quercétine, l'isoquercétine et/ou la rutine ainsi qu'au moins l'une parmi les substances actives de dihydroxyacétone et/ou d'érythrulose sont mélangés successivement avec un véhicule convenable sur le plant cosmétique ou dermatologique.

18. Utilisation non thérapeutique d'une préparation selon l'une ou plusieurs parmi les revendications 7 à 13, pour une application topique ou pour une application à une surface.
